Europäisches Patentamt

European Patent Office

Office européen des brevets

(19)

(11) Veröffentlichungsnummer: **0 242 565**
**B1**

(12) # EUROPÄISCHE PATENTSCHRIFT

(45) Veröffentlichungstag der Patentschrift:
06.06.90

(51) Int. Cl.⁵: **A61B 17/22**

(21) Anmeldenummer: 87103504.4

(22) Anmeldetag: 11.03.87

(54) **Vorrichtung zum Zertrümmern von Steinen.**

(30) Priorität: 25.03.86 DE 8608200 U

(43) Veröffentlichungstag der Anmeldung:
28.10.87 Patentblatt 87/44

(45) Bekanntmachung des Hinweises auf die Patenterteilung:
06.06.90 Patentblatt 90/23

(84) Benannte Vertragsstaaten:
DE FR GB NL

(56) Entgegenhaltungen:
EP-A- 0 133 665
DE-A- 3 429 939
GB-A- 2 102 657
GB-A- 2 140 693

Die Akte enthält technische Angaben, die nach dem
Eingang der Anmeldung eingereicht wurden und die
nicht in dieser Patentschrift enthalten sind.

(73) Patentinhaber: Siemens Aktiengesellschaft,
Wittelsbacherplatz 2, D-8000 München 2(DE)

(72) Erfinder: Schindler, Reinhard, Adlerstrasse 3,
D-8520 Erlangen(DE)

## Beschreibung

Die Erfindung betrifft eine Vorrichtung zum berührungslosen Zertrümmern von Steinen im Körper eines Lebewesens, die einen Stoßwellenerzeuger aufweist, der ein starres, einen Hohlraum umschließendes Gehäuse besitzt, das an seinem einen Ende mit einer Stoßwellenquelle versehen ist und an seinem anderen Ende von einer ein Abstrahlglied bildenden flexiblen Membran abgeschlossen ist, wobei der Hohlraum mit einer die Stoßwellen von der Stoßwellenquelle zu der Membran leitenden Flüssigkeit gefüllt ist.

Eine Vorrichtung dieser Art ist in der EP-A 0 133 665 beschrieben. Diese Vorrichtung ist mit der Membran an der Körperoberfläche im Bereich des zu zertrümmernden Steines, z.B. Nierensteines, anlegbar. Die erzeugten Stoßwellen werden dabei auf den Stein fokussiert und zertrümmern diesen, so daß er auf natürlichem Wege abgeht. Die Flüssigkeit dient als Ausbreitungsmedium, in dem sich die Stoßwelle nach genügend langer Laufzeit ausbildet.

Als Flüssigkeit im Stoßwellenerzeuger kann Wasser benutzt werden. In diesem Fall ist es erforderlich, das Wasser von Zeit zu Zeit zu wechseln und auch ständig zu entgasen, da Gaseinschlüsse die Übertragungseigenschaften verschlechtern würden.

Der Erfindung liegt die Aufgabe zugrunde, eine Vorrichtung der eingangs genannten Art so auszubilden, daß ein Wechsel der Flüssigkeit im Stoßwellenerzeuger oder eine ständige Entgasung entfallen kann, daß also eine Flüssigkeitsfüllung über lange Zeit unverändert benutzt werden kann.

Diese Aufgabe ist erfindungsgemäß dadurch gelöst, daß als Flüssigkeit in dem Hohlraum ein Hydro-Gel benutzt ist. Ein für diesen Zweck geeignetes Hydro-Gel ist z.B. in der Zeitschrift "Spektrum der Wissenschaften", März 1981, Seiten 78 bis 93 beschrieben. Bei der erfindungsgemäßen Vorrichtung sind demgemäß kein Flüssigkeitswechsel und keine ständige Entgasung der Flüssigkeit erforderlich, so daß alle Armaturen, die für diesen Zweck erforderlich wären, entfallen können.

Es ist zwar bereits bekannt, ein mit Hydro-Gel gefülltes Kissen (DE-A-3 429 939) bzw. einen aus Hydro-Gel bestehenden Körper (GB-A-2 102 657) zwischen dem Abstrahlglied eines Ultraschallgenerators und einem zu untersuchenden Objekt anzuordnen. In beiden Fällen ist jedoch das Hydro-Gel nicht in einem starren Gehäuse eingeschlossen und dient lediglich als Ankoppelmedium zwischen Abstrahlfläche und zu untersuchendem Objekt. Im Falle der Erfindung dient das Hydro-Gel dagegen dazu, Stoßwellen von der Stoßwellenquelle zu dem Abstrahlglied zu leiten.

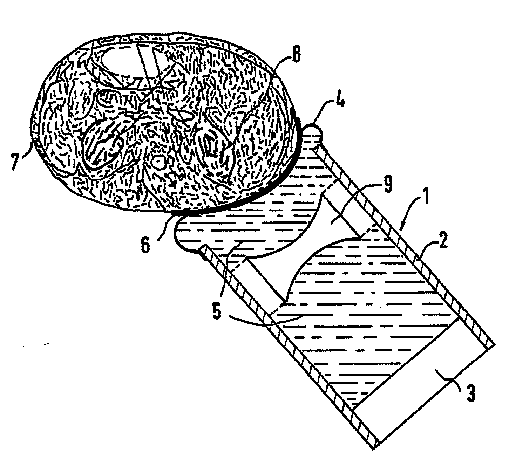

Die Erfindung ist nachfolgend anhand eines in der Zeichnung dargestellten Ausführungsbeispieles näher erläutert.

In der Zeichnung ist ein Stoßwellenerzeuger 1 dargestellt, der aus einem starren Gehäuse 2 besteht, das einen Hohlraum umschließt, der an seinem einen Ende durch eine Stoßwellenquelle 3 und an seinem anderen Ende durch eine flexible Membran 4 abgeschlossen ist. Der Hohlraum des Gehäuses 2 ist mit einem Hydro-Gel 5 gefüllt. Die Applikation des Stoßwellenerzeugers 1 erfolgt derart, daß dieser mit der Membran 4 unter Zwischenschaltung eines Gels 6 an der Körperoberfläche eines im Querschnitt dargestellten Patienten 7 angelegt wird.

Bei dem dargestellten Beispiel soll ein Nierenstein 8 zertrümmert werden. Hierzu werden die von der Stoßwellenquelle 3 erzeugten Stoßwellen mittels des Hydro-Gels 5 zu der Membran 4 geleitet und dabei mit Hilfe einer akustischen Linse 9 auf den Nierenstein 8 fokussiert, so daß die Energie der ausgehend von der Membran 4 durch das Gel 6 in den Körper des Patienten 7 tretenden Stoßwellen auf den Nierenstein 8 konzentriert ist und dessen Zertrümmerung bewirkt. Die akustische Linse weist gestrichelt eingezeichnete Kanäle auf, die den zwischen der Membran 4 und der akustischen Linse 9 befindlichen Abschnitt des Hohlraumes mit dem zwischen der akustischen Linse 9 und der Stoßwellenquelle 3 befindlichen verbinden.

Der Stoßwellenerzeuger 1 wird einmal mit dem Hydro-Gel 2 gefüllt und dann dicht verschlossen. Er kann dann über lange Zeit benutzt werden, ohne daß sich das Hydro-Gel hinsichtlich seiner Übertragungseigenschaften verschlechtert.

## Patentanspruch

Vorrichtung zum berührungslosen Zertrümmern von Steinen (8) im Körper eines Lebewesens (7), die einen Stoßwellenerzeuger (1) aufweist, der ein starres, einen Hohlraum umschließendes Gehäuse (2) besitzt, das an seinem einen Ende mit einer Stoßwellenquelle (3) versehen ist und an seinem anderen Ende von einer ein Abstrahlglied bildenden flexiblen Membran (4) abgeschlossen ist, wobei der Hohlraum mit einer die Stoßwellen von der Stoßwellenquelle (3) zu der Membran (4) leitenden Flüssigkeit (5) gefüllt ist, dadurch gekennzeichnet, daß als Flüssigkeit in dem Hohlraum ein Hydro-Gel (5) benutzt ist.

## Claim

Apparatus for contactless disintegration of calculi (8) in the body of a living being (7), said apparatus having a shock wave generator (1) which has a rigid housing (2) which surrounds a cavity, is provided at its one end with a shock wave source (3) and is sealed at its other end by a flexible membrane (4) forming a radiating element, the cavity being filled with a fluid (5) which conducts the shock waves from the shock wave source (3) to the membrane (4), characterised in that a hydro-gel (5) is used as a fluid in the cavity.

## Revendication

Dispositif de fragmentation sans contact de concrétions (8) dans l'organisme d'un être vivant (7), qui comporte un générateur d'ondes de choc (1) possédant un boîtier (2) rigide, qui entoure une cavité, et dont l'une des extrémités est munie d'une source d'ondes de choc (3) alors que son autre extrémité est fermée par une membrane souple (4) formant un élément rayonnant, la cavité étant emplie d'un liquide (5) dans lequel les ondes de choc se propagent de la source d'ondes de choc (3) à la membrane (4), caractérisé en ce qu'un hydrogel (5) est utilisé comme liquide dans la cavité.